# EUROPEAN PATENT APPLICATION

(11) **EP 2 233 580 A1**
(43) Date of publication of application: **29.09.2010**
(21) Application number: 10155561.3
(22) Date of filing: 04.03.2010
(51) Int. Cl.: C12Q 1/68, H01J 49/16

(54) **Rapid genotyping of SNPs**

(30) Priority: 05.03.2009 US 157812 P
(71) Applicant: The Ohio State University, Columbus OH 43212-1154 (US)
(72) Inventor: Wu, Haifeng M, Columbus, OH 43220 (US); Yang, Shangbin, Columbus, OH 43214 (US); Xu, Lihui, Columbus, OH 43220 (US)
(74) Representative: Boyce, Conor

(57) **Abstract**

Embodiments include a universal and generic method for rapidly genotyping essentially any single nucleotide polymorphism (SNP) and or other polymorphism. Various embodiments include procedures for SNP and/or allele analysis that are easy, cheap, highly multiplexable, easily automatable, and lend themselves to high-throughput. Embodiments are broadly applicable for all applications where SNP determinations are useful.

## Description

### TECHNICAL FIELD

Embodiments relate to systems, methods, and compositions for rapid genotyping of single nucleotide polymorphisms or other polymorphisms.

### BACKGROUND OF THE ART

Over the last 20 years, much knowledge has been gained toward advancing the understanding of human physiology and elucidating the molecular basis of common diseases. There are as many as 3 million single nucleotide polymorphisms (SNPs) in the human genome. A SNP is a single nucleotide variation at a specific location in the genome that, by definition, is found in more than 1% of the population. SNPs are important genetic markers that determine an individual's susceptibility to various diseases. SNPs have been used as molecular markers to evaluate disease processes and to predict patients' drug responses. Because of the importance of SNPs, there exists a need for accurate, rapid, and cost-effective technologies for SNP analysis to advance clinical diagnosis and therapeutics.

### SUMMARY

Embodiments relate to an application of surface-enhanced laser desorption and ionization time-of-flight mass spectrometry (SELDI-TOF MS) for rapidly genotyping SNPs.

Accordingly, embodiments include a method for DNA genotyping a sequence polymorphism of a subject by SELDI-TOF mass spectrometry, comprising the steps of: (a) providing a specimen comprising genomic DNA from the subject; (b) generating from the DNA an allele specific analyte in a reaction mixture; (c) contacting the reaction mixture to a desorption spectrometry target having a chemical affinity to the allele specific analyte; (d) washing the target with an eluant to purify the allele specific analyte; (e) detecting retained analyte on the target by desorption spectrometry; and (f) identifying the genotype for the sequence polymorphism of the subject based on the mass and charge of the allele specific analyte.

Additional embodiments include a method for rapidly genotyping a SNP marker in a DNA sample, comprising the steps of: (a) providing a DNA sample; (b) amplifying a segment of the DNA comprising the SNP marker using a primer pair; (c) performing an oligonucleotide extension using a SNP-specific primer to generate an analyte mixture comprising an allele specific analyte; (d) contacting the analyte mixture to a desorption spectrometry target having a chemical affinity to the allele specific analyte; (e) washing the target with an eluant; (d) detecting retained analyte on the target by desorption spectrometry; and (e) identifying a genotype for the SNP marker in the DNA sample.

Embodiments are also directed to a method for rapidly determining a genotype for a SNP marker of a subject, comprising the steps of: (a) providing a specimen comprising genomic DNA from the subject; (b) amplifying a segment of the DNA comprising the SNP marker using a primer pair; (c) performing an oligonucleotide extension using a SNP-specific primer to generate an analyte mixture comprising an allele specific analyte; (d) contacting the analyte mixture to a desorption spectrometry target having a chemical affinity to the analyte; (e) washing the target with an eluant; (d) detecting retained analyte on the target by desorption spectrometry; and (e) identifying the genotype for the SNP marker of the subject based on the mass and charge of the analyte.

In preferred embodiments, the analyte comprises an oligonucleotide or other nucleic acid.

In some embodiments, the desorption spectrometry target comprises an adsorbent array or matrix. For example, the adsorbent array may comprise a protein chip. In some embodiments, the adsorbent array may comprise a hydrophobic adsorbent, a thiophilic adsorbent, an ion exchange adsorbent, a metal ion adsorbent, or antibody affinity matrices.

In various embodiments, the SNP marker and/or other polymorphism may be associated with a disease such as cancer, cardiovascular disease, thrombotic diseases, autoimmune disease, viral infection, Alzheimer's disease, and diabetes.

In exemplary embodiments, DNA sample or specimens may be obtained from tissue, blood, urine, stool, lymph, cerebrospinal fluid, saliva, buccal swab, and interarticular fluid. In various embodiments, the DNA sample is obtained from a pathological cell.

In a preferred embodiment, the detecting step is performed using a SELDI-TOF mass spectrometer.

An exemplary embodiment comrpises a kit including: a target having a chemical affinity toward oligonucleotides or other nucleic acids; a DNA polymerase; and instructions for performing methods described herein.

### DESCRIPTION OF THE SEQUENCES

The various embodiments can be more fully understood from the following detailed description and the accompanying sequence descriptions, which form a part of this application.

The following sequences conform with 37 C.F.R. 1.821-1.825 ("Requirements for Patent Applications Containing Nucleotide Sequences and/or Amino Acid Sequence Disclosures-the Sequence Rules") and are consistent with World Intellectual Property Organization (WIPO) Standard ST.25 (1998) and the sequence listing requirements of the EPO and PCT (Rules 5.2 and 49.5(a-bis), and Section 208 and Annex C of the Administrative Instructions). The symbols and format used for nucleotide and amino acid sequence data comply with the rules set forth in 37 C.F.R. §1.822.

**Table 1.**

| **SEQ ID NO:** | **Primer** | **Sequence** |
|---|---|---|
| 1 | VKORC1-Fw | GGGTAGGTGCAACAGTAAGG |
| 2 | VKORC1-Rv | TTTGGACTACAGGTGCCTGC |
| 3 | CYP2C9*2-Fw | CCCTCATGACGCTGCGGAAT |
| 4 | CYP2C9*2-Rv | GAAAGATATGGCCACCCCTG |
| 5 | CYP2C9*3-Fw | CATGCAAGACAGGAGCCACA |
| 6 | CYP2C9*3-Rv | TCTCTCACCCGGTGATGGTA |
| 7 | VKORC1-Ext | AAACAACCATTGGCC |
| 8 | CYP2C9*2-Ext | GGGCTTCCTCTTGAACAC |
| 9 | CYP2C9*3-Ext | GCTGGTGGGGAGAAGGTCAA |
| 10 | Prothrombin-Fw | ATGGGGTGAAGGCTGTGACC |
| 11 | Prothrombin-Rv | AGCACTGGGAGCATTGAG |
| 12 | F V Leiden-Fw | ACATCGCCTCTGGGCTAATA |
| 13 | F V Leiden-Rv | TTGAAGGAAATGCCCCATTA |
| 14 | MTHFR-Fw | CCAAAGGCCACCCCGAAG |
| 15 | MTHFR-Rv | GAAAGATCCCGGGGACGATG |
| 16 | Prothrombin-Ext | AAAGTGACTCTCAGC |
| 17 | F V Leiden-Ext | TTTTTTTTTGATCCCTGGACAGGC |
| 18 | MTHFR-Ext | GCTGCGTGATGATGAAATCG |

Table 1 contains examples of primers and primer sequences that can be used for PCR amplification of selected oligonucleotide analytes (SNPs). However, it is appreciated that alternative primers may be designed to address the same oligonucleotide analytes. Furthermore, many other SNPs and/or alleles can be detected with this technology but corresponding primers are not illustrated in this table.

### BRIEF DESCRIPTION OF THE DRAWINGS

A better understanding of the embodiments will be obtained from a reading of the following detailed description and the accompanying drawings in which:

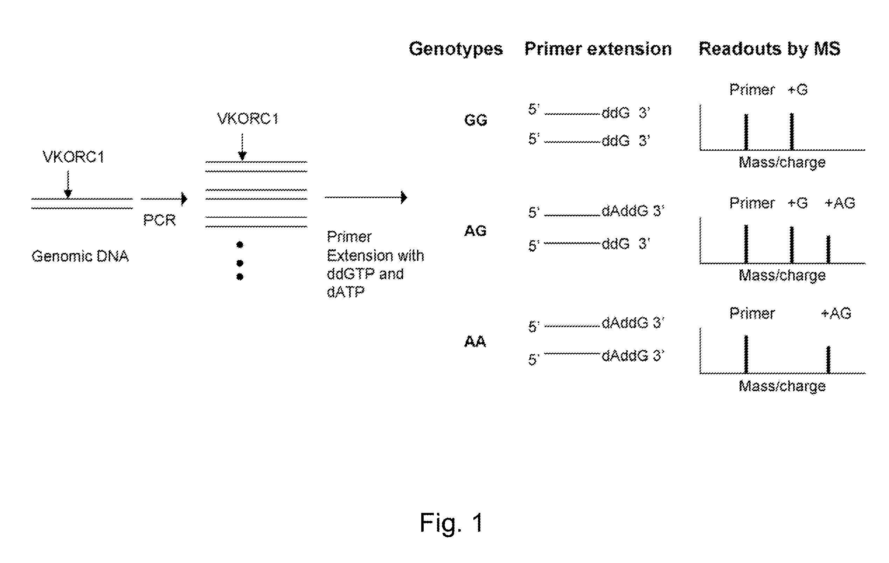

**Figure 1****.** Illustration of the detection of VKORC1 genotype by mass spectrometry (MS). The letter in the panel on the left refers to which bases were added to the primer during the reaction of primer extension. For example, in the 3^{rd} reaction, the primer based extension product was primer extended by dATP and then stopped after addition of ddGTP. Patient genotype at the VKORC1 in this case is however AA.

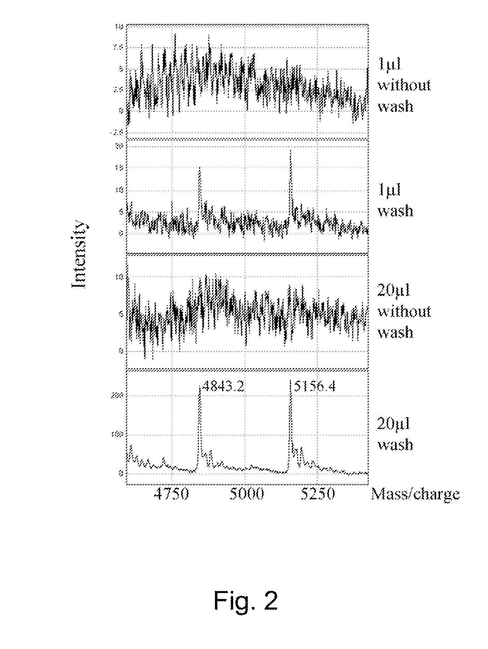

**Figure 2****.** Effect of sample enrichment and washing on the detection of VKORC1 genotype by SELDI-TOF MS. The DNA sample from a patient with GA genotype at *VKORC1 3673G>A* site was subjected to PCR to amplify the VKORC1 SNP. Afterwards, two different volumes of PCR products were applied to Q10 Chips: one of each with no washing step, and one of each washed with 50 mM Tris HCl, pH 7. Finally, the analytes on the Q10 Chips were detected by a SELD-TOF MS.

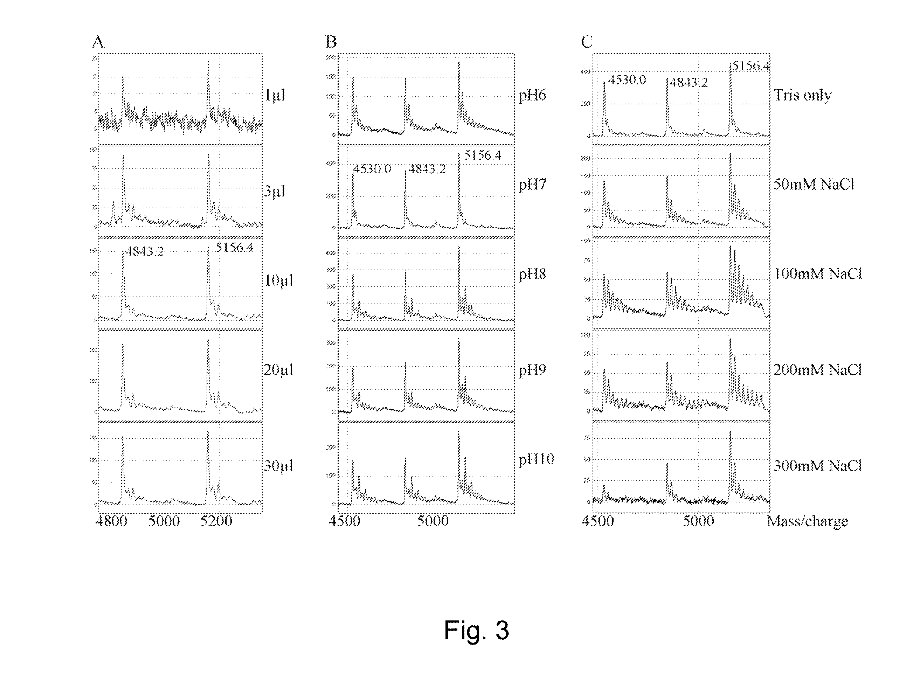

**Figure 3.** A) Dose-dependent enrichment of VKORC1 PCR product by Q10 anionic Chip. PCR was employed to amplify the DNA fragment containing VKORC1 site. Different volumes of PCR products were then loaded onto Q10 anionic Chip. After incubation and washing with 50 mM Tris, pH 7.0, the sample was analyzed by SELDI-TOF MS. This patient has a GA genotype at *VKORC1 3673G>A* site. B) pH dependent binding of PCR product to Q10 Chips. Equal amounts of PCR amplified VKORC1 DNA (20 µl) were applied to Q10 Chips. Each Chip was subject to a washing step using a buffer with different pH value, prior to analysis by SELDI-TOF MS. The buffer with pH 6 was phosphate buffer while the buffers with pH values from 7 to 10 were Tris-HCl buffers. C) Effect of salts on the detection of genotype by SELDI-TOF MS. PCR amplified VKORC1 products (20 µl each) were applied to Q10 Chips, followed by washing with 50 mM Tris HCl, pH 7, containing various concentrations of NaCl. Afterwards, the analytes were genotyped by a SELDI-TOF MS.

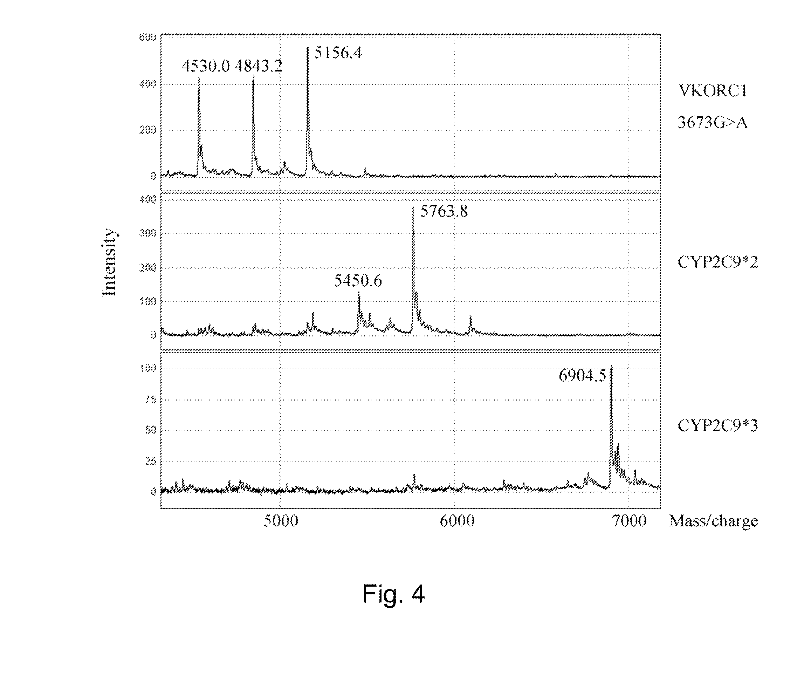

**Figure 4****.** Genotyping SNPs, *VKORC1 3673G>A, CYP2C9*2* and *CYP2C9*3,* individually by SELDI-TOF MS. The DNA sample from one patient was genotyped for SNPs at the *VKORC1 3673G>A, CYP2C9*2*, and *CYP2C9*3* sites. Both PCR reactions and the analysis by MS were performed separately for each SNP.

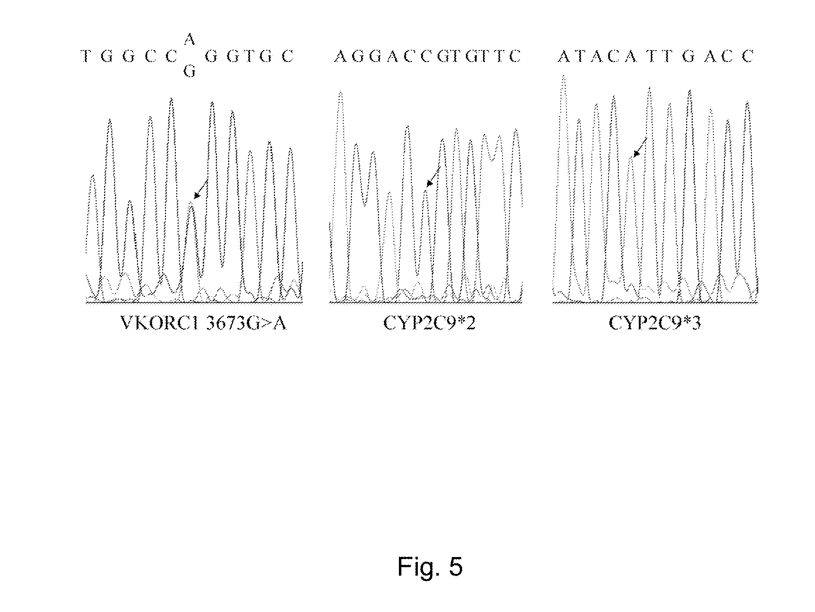

**Figure 5****.** Confirming genotyping results by DNA sequencing. The product from each PCR assay was subject to the BigDye Terminator Reaction Chemistry v3.1 for sequence analysis on Applied Biosystems 3730 DNA Analyzers.

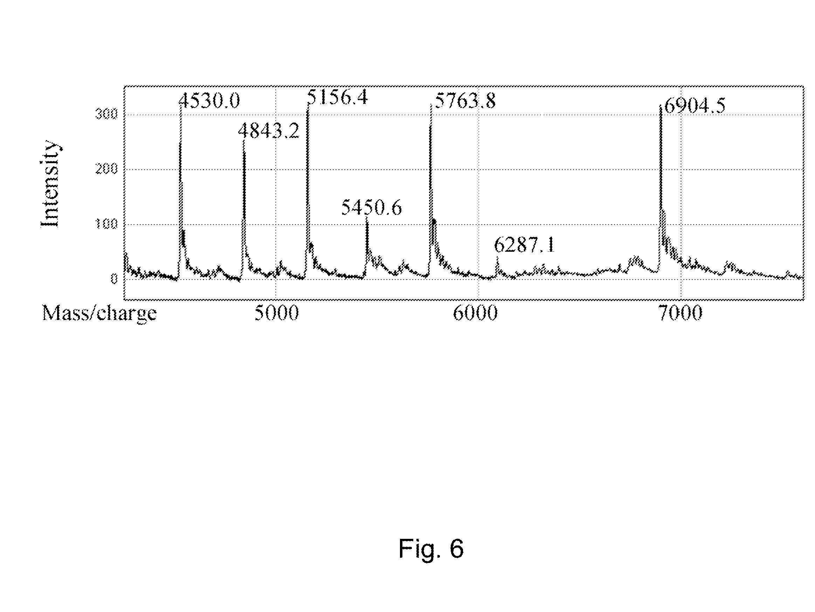

**Figure 6****.** Multiplexed Genotyping of three SNPs, *VKORC1 3673G>A, CYP2C9*2* and *CYP2C9*3.* PCR amplification for each SNP was performed separately on DNA from the subject shown in Figure 4. PCR products for three SNPs were pooled and applied onto Q10 Chip, followed by washing and genotyping by SELDI-TOF MS.

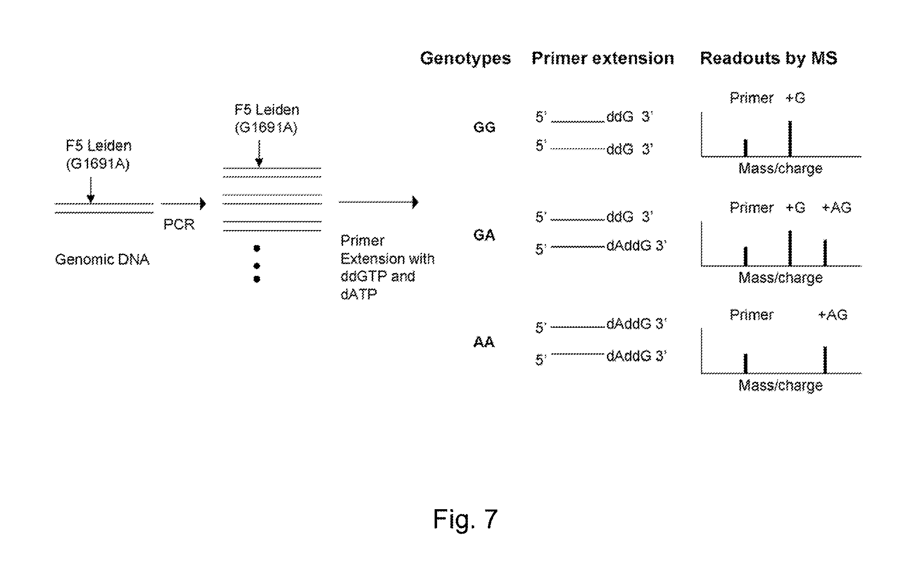

**Figure 7****.** Illustration on the detection of factor V Leiden (G1691A) genotype by mass spectrometry.

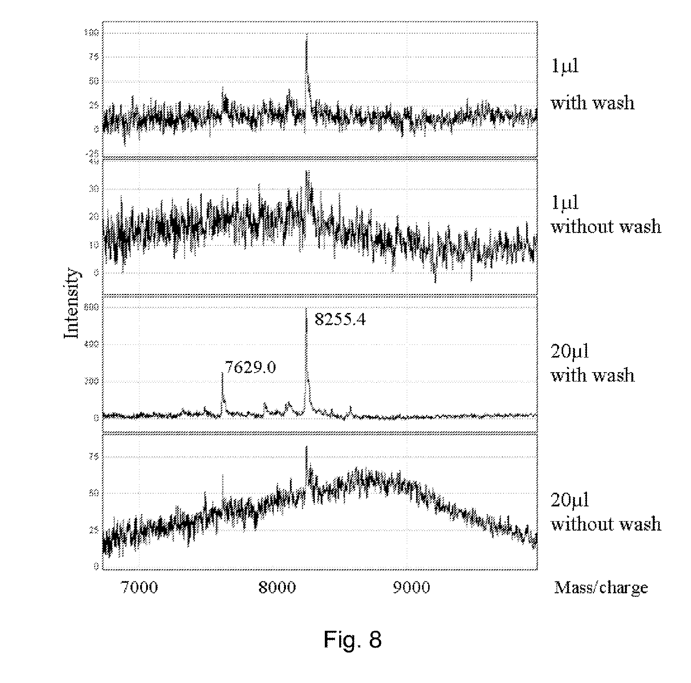

**Figure 8****.** Effect of sample enrichment and washing on the detection of the genotype at factor V Leiden (G1691A) by mass spectrometry. The DNA sample from a patient with known GA genotype at factor V Leiden was subjected to PCR to amplify the SNP. Afterwards, two different volumes of PCR products were applied to Q10 Chips: one with no washing step, and one washed with 50 mM Tris HCl, pH 7. Finally, the analytes on the Q10 Chips were detected by a SELD-TOF mass spectrometer.

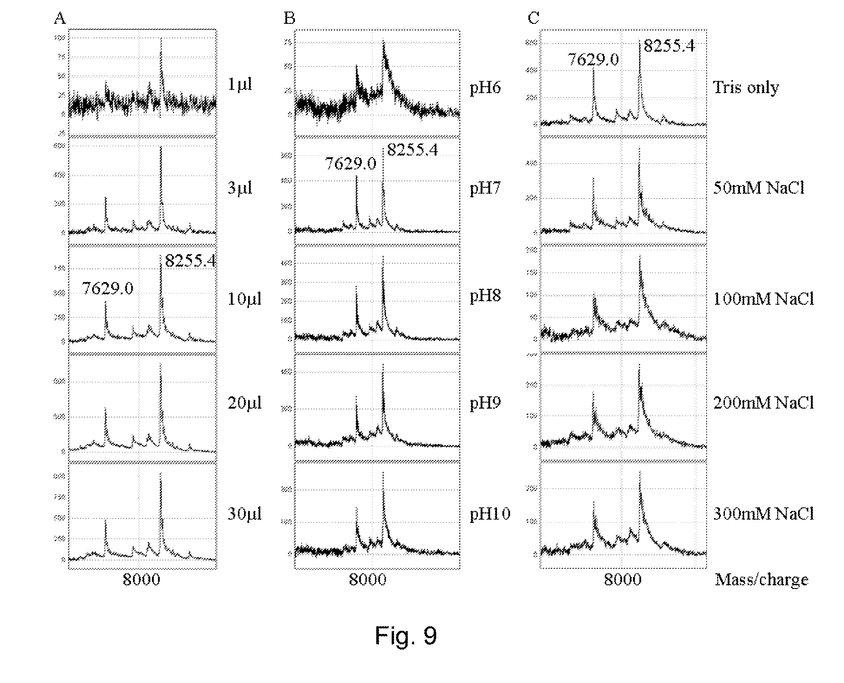

**Figure 9****.** Determination of the optimal sample loading volume, pH buffer, and washing condition for genotyping factor V Leiden by SELDI-TOF mass spectrometer. This study subject has a GA genotype at factor V Leiden (G1691A).

Panel A: Dose-dependent enrichment of factor V Leiden (G1691A) PCR product by Q10 anionic Chip. Panel B: pH dependent binding of PCR product to Q10 Chips. Equal amounts of PCR amplified DNA (20 µl) containing factor V Leiden SNP were applied to Q10 Chips, followed with a washing step using a buffer with a different pH value. The buffers with pH 5 and 6 were phosphate buffers while the buffers with pH values from 7 to 10 were Tris-HCl buffers. Panel C: Effect of salts on the detection of genotype by SELDI-TOF. 20 µl PCR amplified factor V Leiden SNP were applied to Q10 Chips, followed by washing with 50 mM Tris HCl, pH 7, containing various concentrations of NaCl.

**Figure 10****.** Genotyping SNPs, prothrombin G20210A, factorV Leiden, and MTHFR C677T, individually by SELDI-TOF MS. The DNA sample from one patient was genotyped for SNPs at the prothrombin G20210A, factor V Leiden, and MTHFR C677T sites. Both PCR reactions and the analysis by mass spectrometer were performed separately for each SNP.

**Figure 11****.** Confirming genotyping results by DNA sequencing. The product from each PCR assay was subject to the BigDye Terminator Reaction Chemistry v3.1 for sequence analysis on Applied Biosystems 3730 DNA Analyzers.

**Figure 12****.** Multiplexed Genotyping of three SNPs, prothrombin G20210A, factor V Leiden, and MTHFR C677T. PCR amplification for each SNP was performed separately on DNA from the subject shown in Figure 6. PCR products for three SNPs were pooled, applied onto Q10 Chip, followed by washing and genotyping by SELDI-TOF mass spectrometer.

**Figure 13****.** Illustration of the detection of CYP2D6*3 (A2549 del) by SELDI-TOF.

**Figure 14****.** Illustration of the detection of UGT1A1 TA repeats by SELDI-TOF.

### DETAILED DESCRIPTION

Embodiments include a universal and generic method for rapidly genotyping essentially any SNP. Various embodiments include procedures for SNP and/or allele analysis that are easy, cheap, highly multiplexable, easily automatable, and lend themselves to high-throughput. Embodiments are broadly applicable for all applications where SNP determinations are useful. For example, embodiments permit rapid SNP determinations at multiple regions of the genome of an individual or subject, in order to determine, for example, susceptibility to disease. Embodiments may also be applied to SNPs that influence the efficacy and safety profiles of many drug therapies.

In various embodiments, allele specific analytes are generated in an analyte mixture. In some embodiments, the allele specific analytes are generated by providing a DNA sample; amplifying a segment of the DNA comprising the SNP marker using a primer pair; and performing an oligonucleotide extension using a SNP-specific primer to generate an analyte mixture comprising an analyte. In alternative embodiments, other methods for generating allele specific analytes may be used (see e.g., US Patent Application Publication No. 2004/0038234). Examples include PCR based amplifications of specific alleles having a variable number of tandem repeats.

In various embodiments, the analyte mixture may then be contacted to a desorption spectrometry target having a chemical affinity to the analyte. The target may then be washed with an eluant and retained analytes on the target may be detected by desorption spectrometry. Based on the corresponding mass spectrometry readout, a genotype for an SNP marker or other allele may thus be assigned.

Unless otherwise defined, all technical and scientific terms used herein have the same meaning as commonly understood by one of ordinary skill in the art to which these embodiments pertain. Although methods and materials similar or equivalent to those described herein can be used in the practice or testing of various embodiments, suitable methods and materials are described below. In case of conflict, the present specification, including definitions, will control. In addition, the materials, methods, and examples are illustrative only and not intended to be limiting.

The section headings used herein are for organizational purposes only and are not to be construed as limiting the described subject matter in any way. It will be appreciated that there is an implied "about" prior to metrics such as temperatures, concentrations, and times discussed in the present teachings, such that slight and insubstantial deviations are within the scope of the present teachings herein. In this application, the use of the singular includes the plural unless specifically stated otherwise. Also, the use of "comprise", "comprises", "comprising", "contain", "contains", "containing", "include", "includes", and "including" are not intended to be limiting. It is to be understood that both the foregoing general description and the following detailed description are exemplary and explanatory only and are not restrictive of the invention. The articles "a" and "an" are used herein to refer to one or to more than one (i.e., to at least one) of the grammatical object of the article. By way of example, "an element" means one element or more than one element.

SELDI-TOF MS: SELDI-TOF MS refers to surface-enhanced laser desorption and ionization time-of-flight mass spectrometry (SELDI-TOF MS). The surface-enhanced laser desorption and ionization mass spectrometer (SELDI-TOF MS) is a unique type of MS that involves matrix array technology. Different matrices or chips are available to isolate or enrich a specific analyte before mass analysis. Several kinds of chips are available, each coated with a specific chemical matrix. Examples include immobilized metal affinity capture (IMAC) chips, cation or anion exchange chips, or chips with hydrophobic properties. Therefore, it is possible to pre-select a chip (based on the properties of target analytes), enrich a target molecule, remove unwanted elements such as salt, and then detect it using a MS.

Target: target refers to a chip or other surface which has been modified to achieve biochemical affinity with an analyte compound.

Polymorphism: polymorphism refers to the presence of two or more variants of a nucleotide sequence in a population. A polymorphism may comprise one or more base changes, an insertion, a repeat, or a deletion. A polymorphism includes e.g. a simple sequence repeat (SSR) and a single nucleotide polymorphism (SNP), which is a variation, occurring when a single nucleotide: adenine (A), thymine (T), cytosine (C) or guanine (G)--is altered. SNPs make up e.g. 90% of all human genetic variations, and occur about every 100 to 300 bases along the human genome. Two of every three SNPs substitute Cytosine (C) with Thymine (T). Variations in the DNA sequences of e.g. humans or plants can affect how they handle diseases, bacteria, viruses, chemicals, drugs, etc.

Nucleic acid: a nucleic acid may include any polymer or oligomer of pyrimidine and purine bases, preferably cytosine, thymine, and uracil, and adenine and guanine, respectively (See Albert L. Lehninger, Principles of Biochemistry, at 793-800 (Worth Pub. 1982)). The present invention contemplates any deoxyribonucleotide, ribonucleotide or peptide nucleic acid component, and any chemical variants thereof, such as methylated, hydroxymethylated or glycosylated forms of these bases, and the like. The polymers or oligomers may be heterogenous or homogenous in composition, and may be isolated from naturally occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

SNP marker: a SNP marker is a marker that is based on an identified single nucleotide polymorphism at a certain position.

As used herein, the term "subject" is intended to include humans, non-human animals, and plants. The term "non-human animals" includes all vertebrates, e.g., mammals and non-mammals, such as non-human primates, pigs, chickens and other birds, mice, dogs, cats, cows, and horses.

Sequencing: The term sequencing refers to determining the order of nucleotides (base sequences) in a nucleic acid sample, e.g. DNA or RNA.

Primers: in general, the term primers refer to DNA strands which can prime the synthesis of DNA. DNA polymerase cannot synthesize DNA de novo without primers: it can only extend an existing DNA strand in a reaction in which the complementary strand is used as a template to direct the order of nucleotides to be assembled. Herein, the synthetic oligonucleotide molecules which are used in a polymerase chain reaction (PCR) are referred to as primers. An "SNP-specific primer" is a primer appropriate for oligonucleotide extension at an SNP marker.

DNA amplification: the term DNA amplification will be typically used to denote the in vitro synthesis of double-stranded DNA molecules using PCR. It is noted that other amplification methods exist and they may be used without departing from the gist.

In various embodiments, DNA is to be provided. This can be done by methods known in the art per se. The isolation of DNA is generally achieved using common methods in the art such as the collection of tissue from a member of the population, DNA extraction (for instance using the Q-Biogene fast DNA kit), quantification and normalisation to obtain equal amounts of DNA per sample. The DNA can be from a variety of sources (Genomic, RNA, cDNA, BAc, YAC etc.) and organisms (human, mammal, plant, microorganisms, etc.). The isolated DNA may be pooled.Also provided are various kits for performing the methods provided herein. The kits provided herein may comprise a target having a chemical affinity toward oligonucleotides or other nucleic acids. In various embodiments, the kits may also comprise a DNA polymerase. Additionally, the kit may include instructional materials for performing various methods presented herein. These instructions may be printed and/or may be supplied, without limitation, as an electronic-readable medium, such as a floppy disc, a CD-ROM, a DVD, a Zip disc, a video cassette, an audiotape, and a flash memory device. Alternatively, instructions may be published on an internet web site or may be distributed to the user as an electronic mail. When a kit is supplied, the different components can be packaged in separate containers. Such packaging of the components separately can permit long term storage without losing the active components' functions.

EXAMPLES

The following examples are included to demonstrate embodiments. It should be appreciated by those skilled in the art that the techniques disclosed in the examples which follow represent techniques discovered by the inventors to function well in practice. However, those of skill in the art should, in light of the present disclosure, appreciate that many changes can be made in the specific embodiments which are disclosed and still obtain a like or similar result without departing from the the invention. More specifically, it will be apparent that certain agents that are both chemically and physiologically related may be substituted for the agents described herein while the same or similar results would be achieved. All such similar substitutes and modifications apparent to those skilled in the art are deemed to be within the scope of the invention.

General Methods

Generally, nomenclatures utilized in connection with, and techniques of, cell and tissue culture, molecular biology, and protein and oligo- or polynucleotide chemistry and hybridization described herein are those well known and commonly used in the art. Standard techniques are used, for example, for nucleic acid purification and preparation, chemical analysis, recombinant nucleic acid, and oligonucleotide synthesis. Enzymatic reactions and purification techniques are performed according to manufacturer's specifications or as commonly accomplished in the art or as described herein. The techniques and procedures described herein are generally performed according to conventional methods well known in the art and as described in various general and more specific references that are cited and discussed throughout the instant specification. See, e.g., Sambrook et al., Molecular Cloning: A Laboratory Manual (Third ed., Cold Spring Harbor Laboratory Press, Cold Spring Harbor, N.Y. 2000). The nomenclatures utilized in connection with, and the laboratory procedures and techniques of described herein are those well known and commonly used in the art.

**A. Genotyping of SNPs Influencing Warfarin Drug Response**

Warfarin (Coumadin) is an anticoagulant that disrupts the process of vitamin K recycling. Vitamin K is an essential cofactor for the post-translational modification of several clotting factors including Factors II, VII, IX and X, and the anticoagulant proteins C and S. Patients on therapeutic warfarin exhibit low clotting potential and therefore show less propensity for thrombotic disorders. There are two major pathways that determine warfarin drug efficacy. First, warfarin inhibits the function of vitamin K epoxide reductase (VKORC1) that is responsible for regeneration and recycling of vitamin K. Suppression of VKORC1 results in a reduction of endogenous vitamin K and a subsequent reduction of vitamin K-dependent clotting factors. It has been shown that *VKORC1* variants exhibit different sensitivities to the drug. Patients who are found to have the genotype AA for the SNP *VKORC1 3673G>A* in the promoter region, have a lower amount of VKORC1, and therefore typically require a lower warfarin dose than average. In contrast, those who are GG genotype are resistant to warfarin, typically requiring a greater dose of the drug to achieve desired therapeutic effect. Other patients carry an AG genotype. Secondly, the elimination of warfarin is almost entirely controlled through drug metabolism. Warfarin drug effect is primarily removed when it is converted to a hydroxylated metabolite by hepatic microsomal enzymes (cytochrome P-450). The cytochrome P-450 2C9 (CYP2C9) isozyme appears to be the principal form of human liver P-450, which controls the drug activity of warfarin. The variant alleles, particularly variant *CYP2C9*2* and *CYP2C9*3,* result in decreased hydroxylation of warfarin, therefore reducing the rate of warfarin clearance. The role of genotype based warfarin therapy to improve both drug efficacy and safety has been implicated in several recently studies.

Embodiments comprise novel methods and systems for rapidly genotyping SNPs. In the following examples, an embodiment was used to successfully genotype SNPs known to influence warfarin sensitivity or warfarin metabolism.

**(i) PCR Amplification of SNPs and PCR product cleanup**

PCR was conducted to amplify DNA fragments containing each SNP site. Each PCR mixture (20 µl) consisted of 18 µl of PCR SuperMix (Invitrogen Cat.No. 10572-014), forward and reverse PCR primers at 1 µM, and 50ng of genomic DNA (extracted from whole blood with DNeasy Tissue Kit QIAGEN Cat.No. 69504) as the template. The sequence of primers and expected lengths of PCR products are shown in Table 2.

| **Table 2: PCR primers used to amplify the target SNPs** | | |
|---|---|---|
| Name | Sequence | Product Length |
| VKORC1-Fw | 5' GGGTAGGTGCAACAGTAAGG 3' | 236bp |
| VKORC1-Rv | 5' TTTGGACTACAGGTGCCTGC 3' | |
| CYP2C9*2-Fw | 5' CCCTCATGACGCTGCGGAAT 3' | 195bp |
| CYP2C9*2-Rv | 5' GAAAGATATGGCCACCCCTG 3' | |
| CYP2C9*3-Fw | 5' CATGCAAGACAGGAGCCACA 3' | 230bp |
| CYP2C9*3-Rv | 5' TCTCTCACCCGGTGATGGTA 3' | |

The final optimized reaction consisted of denaturation at 94°C for 3 minutes, followed by 35 cycles of 94°C for 20 seconds, 56°C for 20 seconds, and 72°C for 20 seconds, with a final extension at 72°C for 5 minutes. After PCR, PCR primers and dNTPs in the PCR product (11.5 µl) were removed by adding a polishing mixture consisting of 1 U of Shrimp Alkaline Phosphatase (USB Product No. 70092Y) and 10 U of Exonuclease I (USB Product No.70073X). This reaction was incubated for 40 minutes at 37°C followed by a step of inactivation at 95°C for 15 minutes.

**(ii) Oligonucleotide extension of the PCR products**

A SNP-specific primer was used to perform oligonucleotide extension for each PCR product. The reaction contained the polished PCR product, 2.5 U of Diamond DNA polymerase (Bioline, Cat. No. BIO21058), 0.17 µM genotyping primer, 5 mM MgCl₂ solution, and 6.7 µM specific ddNTP and dNTP respectively. The reaction consisted of 50 cycles of 94°C for 20 seconds, 37°C for 20 seconds and 72°C for 20 seconds. The SNP-specific primers and the masses of products are listed in Table 3.

| **Table 3: primers for oligonucleotide extension (genotyping) and the theoretical masses of the primers and their extension products** | | | | |
|---|---|---|---|---|
| SNPs | rs# in NCBI SNP database | Genotyping Primers and Theoretical Mass | Genotypes | Theoretical Mass of the Product (Da) |
| VKORC1 3673G>A | rs9923231 | 5' AAACAACCATTGGCC 3', 4530.0Da | GG | 4843.2 |
| | | | GA | 4843.2 & 5156.4 |
| | | | AA | 5156.4 |
| CYP2C9*2 455C>T | rs1799853 | 5' GGGCTTC CTCTTGAACAC 3', 5450.6Da | CC | 5763.8 |
| | | | CT | 5763.8 & 6077.0 |
| | | | TT | 6077.0 |
| CYP2C9*3 1100A>C | rs1057910 | 5' GCTGGTGGGGAGAAGGTCAA 3', 6287.1Da | AA | 6904.5 |
| | | | AC | 6600.3 & 6904.5 |
| | | | CC | 6600.3 |

**(iii) Detection of the primer extension product by SELDI-TOF MS**

The primer oligo base extension products (30 µl) were applied to the proteinChips (Vermillion, Inc) assembled in a bioprocessor. Because the SNPs containing DNA products from PCR reactions are negatively charged, we chose a Q10 anionic chip to specifically enrich and clean up the SNP analytes in this study. After incubation for 15 minutes, the chips were washed by a buffer containing 50 mM Tris, pH 7.0, three times. One µl of matrix containing 0.25 M 3-Hydroxypicolinic acid (3-HPA) and 0.03 M diammonium citrate in 10% acetonitrile was added to each spot on the chip, and allowed to air dry. Finally, the chips were analyzed by PCS4000 SELDI-TOF MS (Vermillion, Inc).

**(iv) Analytical validation of genotyping accuracy by SELDI-TOF MS**

Sixty three randomly collected clinical samples were tested by SELDI-TOF MS to determine the genotypes at the three SNP sites, *VKORC1 3673G>A, CYP2C9*2* and *CYP2C9*3,* resulting in a total of 189 genotype data points. Afterwards, the same set of the samples were genotyped in the clinical reference laboratory at the Ohio State University Medical Center using an FDA approved platform INFINITI Analyzer (Autogenomics, Carlsbad CA). Additionally, PCR products (n=189) that were used for genotyping all three SNPs from 63 study subjects by SELDI-TOF MS were sequenced bidirectionally to confirm the accuracy of the genotypes. Bidirectional sequencing was carried out by the Nucleic Acid Shared Resource of the Comprehensive Cancer Center, the Ohio State University using specific primers up and down stream of each SNP site.

**(v) Results**

By using VKORC1 as an example, Figure 1 illustrates the principle by which SNPs are genotyped by a MS. The DNA containing the SNP *VKORC1 3673G>A* was first amplified by a PCR reaction. The amplified product is then used as the template for primer based extension, giving rise to DNA fragments with specific lengths corresponding to their genotypes. Afterwards, the genotypes are determined by MS based on the masses of PCR products.

Optimization of the test conditions is essential to achieve test precision, detection sensitivity, and test reliability. First, various conditions for the PCR reactions, including the number of amplification cycles, annealing temperature, and extension time were optimized (data not shown). This enabled us to maximize the PCR product (analyte) and at same time reduce the test turnaround time. Afterwards, efforts were made to optimize the conditions for the detection process. For a typical readout by a routine MS such as Matrix-assisted laser desorption/ionization time of flight (MALDI-TOF), approximately 1 µl of sample can be applied to a plate. After the sample is dried out on the plate, the matrix is added followed by readout on a MS. In contrast, the bioprocessor in SELDI-TOF MS allows for loading as much as 500 µl of sample to be concentrated on a chip surface. The bound sample can then be subjected to a clean-up to remove unwanted components before detection.

Figure 2 describes the properties of the on-chip sample enrichment and clean-ups by SELDI-TOF MS in comparison to a condition mimicking the performance by MALDI-TOF MS. The PCR product containing *VKORC1 3673G>A* was measured under 4 different conditions on Q10 chips. The top two readouts detect the analyte derived from 1 µl PCR sample, the loading volume mimicking the capacity when detected by MALDI-TOF MS. In order to examine the on-chip clean-up effect, one of the two readouts was subjected to washing (50 mM Tris HCl, pH 7.0) prior to detection. The other two readouts were obtained after on-chip enrichment of 20 µl PCR products, with or without an additional washing step. The results demonstrate that SELDI-TOF MS offers remarkable sample concentrating properties, greatly improving test detection capability. It is also apparent that a washing step is necessary in both procedures to enhance signal to noise ratios. Likely, the washing step after the sample was loaded onto the plate removed interfering substances in the PCR products and thus, greatly enhanced detection of the analyte by MS.

Next, we performed analysis to determine the optimal conditions for genotyping VKORC1 by SELDI-TOF MS. As shown in figure 3A. Q10 chip captures the PCR product containing VKORC1 in a dose-dependent manner. When various volumes of the PCR products were applied to the Q10 chips, the amount of the analyte detected by SELDI-TOF MS corresponds to the amount loaded onto the chip until the binding capacity is reached. In Figure 3B, we evaluated the pH dependency for the binding of the analyte on the Q10 chip. The PCR product (20 µl) was loaded onto each of the Q10 chips using a bioprocessor. After 15 minutes of binding reaction, Q10 chips were each washed 3 times with buffers of different pHs. As a result, the buffer with a pH of 7 appears to offer the optimal binding of analyte to Q10 chips with respect to the signal/noise ratios and peak readout, while buffers with either alkaline or acidic pH conditions significantly reduced the binding of analytes to the chip in a pH dependent manner. Finally, we examined the effect of various salt concentrations on the measurement of DNA analyte by SELDI-TOF MS. After on-chip sample enrichment, the chips were washed using washing buffers with different concentrations of NaCl as shown in Figure 3C. As expected, the buffer with lowest salt concentration gives rise to the cleanest analyte peak. When a high concentration of salt is used, the Na⁺ adducts, a cluster of side peaks with a difference of 22 Daltons in molecular weight, are seen on the right side of the analyte peak.

When using the optimized conditions for PCRs and the detection process, all known SNPs that influence drug sensitivity, *VKORC1 3673G>A, CYP2C9*2* and *CYP2C9*3,* were adequately detected by SELDI-TOF MS. Representative mass spectrums for these three SNPs are shown in Figure 4. The masses of the peaks for each of the three SNPs are solid matches with the theoretical masses shown in Table 3. For instance, the top panel in Figure 4 is used to genotype *VKORC1 3673G>A.* SELDI-TOF MS detected three peaks with masses of 4530.0 Da, 4843.2 Da and 5156.4 Da. Based on Table 3, the genotype for this individual at *VKORC1 3673G>A* SNP is heterozygous GA. Similarly, the genotype for this individual at SNP *CYP2C9*2* and SNP *CYP2C9*3* are determined to be CC and AA homozygote respectively.

In order to evaluate the accuracy of the genotyping by SELDI-TOF MS, the PCR products (n=189, corresponding to a sum of 3 SNPs from 63 clinical samples) that were genotyped by SELDI-TOF MS were all sequenced bidirectionally. Figure 5 illustrates the site of sequences that determine the genotype at three different SNPs by DNA sequencing. The results demonstrated that the genotype determined by SELDI-TOF MS agreed 100% with the sequencing data at all three SNPs of VKORC1 3673G>A, CYP2C9*2 and CYP2C9*3. Additionally, the test accuracy by SELDI-TOF MS has been validated by comparing the result to the reports from the clinical laboratory using an FDA approved platform (INFINITI Analyzer, Autogenomics). Sixty three study subjects were genotyped for 3 SNPs of *VKORC1 3673G>A, CYP2C9*2* and *CYP2C9*3* by both SELDI-TOF MS and INFINITI analyzer. When comparing the genotyping results between SELDI-TOF and the INFINITI, the overall agreement rate was 98.94% (187/189). For the two genotypes that disagreed, the results by SELDI-TOF MS match the bidirectional sequencing data.

In order to improve the genotyping efficiency by SELDI-TOF MS, we designed different length of primers to amplify the DNA fragment at different SNP sites. As a result, PCR products containing different SNPs (*VKORC1 3673G>A, CYP2C9*2* and *CYP2C9*3)* vary approximately by 1000 Daltons in mass (see Table 3). Following PCR amplification, all three PCR products, detected separately in Figure 4, were loaded onto the same spot on the Q10 chip, and then analyzed by MS. As shown in Figure 6, all three SNPs were accurately genotyped with the masses of the analytes corresponding exactly to the masses shown in Figure 4 when SNPs were detected individually. *We have applied multiplexed genotyping to all 63 study subjects. The genotype results from multiplex detection agreed 100% with the results when PCR extension products were individually genotyped by SELDI-TOF MS*. The ability to simultaneously detect all SNPs on the same chip has helped to reduce the overall test turnaround time from sample collection to the test reporting to less than five hours.

**B. Genotyping for Hereditary Thrombophilia**

Venous thromboembolism (VTE) is associated with significant morbidity and mortality. The most common predisposing clinical factors for VTE are advanced age, immobilization, pregnancy, oral contraception, or cancer. However, in many cases, VTE occurs at younger age or at an unusual site, without apparent clinical risk factors. These idiopathic cases of VTE are often associated with underlying genetic predisposition(s). Thrombophilia refers to hereditary conditions that predispose patients to the risk of VTE. One type of genetic effect is due to DNA mutations that result in a deficiency of anticoagulant factor including protein C, protein S deficiencies, or antithrombin. When anticoagulation function is impaired, patients are prothrombotic. The prevalence of these mutations however is very low. The second type of hereditary influence is due to single nucleotide polymorphisms (SNPs) that carry at high frequency in the general population. Most commonly indicated SNPs for diagnosis of hereditary thrombophilia are factor V Leiden (G1691A) and prothrombin mutation G20210A. Some studies also suggest the role of the SNP at methylenetetrahydrofolate reductase (MTHFR C677T) in hereditary thrombophilia. The SNPs that predispose patients to thrombosis are found in more than half of all cases of idiopathic VTE. At the present time, genotyping these SNPs along with the functional studies for protein C, protein S and antithrombin are recommended for individuals at a high risk for clotting disorders. These individuals are identified when patients have an early onset of VTE, VTE occurring at an unusual site, a family history of thrombotic disorders, or women with recurrent pregnancy losses. The information on genetic risk factors for thrombosis enables clinicians to more properly diagnose and manage patients.

**(i) PCR Amplification of SNPs and PCR product cleanup**

The DNA from whole blood was extracted using the DNeasy Tissue Kit from Qiagen according to manufacturer's instruction. PCRs were then conducted to amplify DNA fragments containing each SNP site. Each PCR mixture (20 µl) consisted of 18 µl of PCR SuperMix (Invitrogen Cat.No. 10572-014), forward and reverse PCR primers at 1 µM, and 20 ng genomic DNA as template. The sequence of primers and expected length of PCR products are shown in Table 4.

| **Table 4 PCR primers used to amplify the target SNPs** | | |
|---|---|---|
| Name | Sequence | Product Length |
| Prothrombin-Fw | 5' ATG GGG TGA AGG CTG TGA CC 3' | 221bp |
| Prothrombin-Rv | 5' AGC ACT GGG AGC ATT GAG 3' | |
| F V Leiden-Fw | 5' ACA TCG CCT CTG GGC TAA TA 3' | 169bp |
| F V Leiden-Rv | 5' TTG AAG GAA ATG CCC CAT TA 3' | |
| MTHFR-Fw | 5' CCA AAG GCC ACC CCG AAG 3' | 180bp |
| MTHFR-Rv | 5' GAA AGA TCC CGG GGA CGA TG 3' | |

The primers that target MTHFR were designed based on a previous publication. The final optimized reaction consisted of denaturation at 94 °C for 3min, followed by 35 cycles of 94 °C for 20 seconds, 56 °C for 20 seconds and 72°C for 20 seconds, with a final extension at 72°C for 5 minutes. After PCR, PCR primers and dNTPs in the PCR product (11.5µl) were removed by adding a polishing mixture consisting of 1 U of Shrimp Alkaline Phosphatase (USB Product No. 70092Y) and 10 U of Exonuclease I (USB Product No.70073X). This reaction was incubated for 30 minutes at 37 °C followed by a step of inactivation at 85 °C for 20 minutes.

**(ii) Oligonucleotide extension of the PCR products**

SNP-specific primer was used to perform oligonucleotide extension for each PCR product. The reaction contained the polished PCR product, 5 U of Diamond DNA polymerase (Bioline, Cat.No. BIO21058), 0.125 µM genotyping primer, 10 mM MgCl₂ solution, and 16 µM specific ddNTP and dNTP respectively. The reaction consisted of 50 cycles of 94°C for 20 seconds, 37°C for 20 seconds and 72°C for 20 seconds. The SNP-specific primers and the masses of the products are listed in Table 5.

| **Table 5: primers for oligonucleotide extension (genotyping) and the theoretical masses of the primers and their extension products** | | | | |
|---|---|---|---|---|
| SNPs | rs# | Genotyping Primers and Theoretical Mass | Genotypes | Theoretical Mass of the Product (Da) |
| Prothrombin 20210G>A | rs1799963 | 5' AAAGTGACTCTCAGC 3' 4561.0Da | GG | 4874.2 |
| | | | GA | 4874.2 & 5500.7 |
| | | | AA | 5500.7 |
| F V Leiden 1691G>A | rs6025 | 5' TTTTTTTTTGATCCCTGGACAGGC 3' 7315.8Da | GG | 7629.0 |
| | | | GA | 7629.0 & 8255.4 |
| | | | AA | 8255.4 |
| MTHFR 677C>T | rs1801133 | 5' GCTGCGTGATGATGAAATCG 3' 6197.1Da | CC | 6510.3 |
| | | | CT | 6510.3 & 8284.4 |
| | | | TT | 8284.4 |

**(iii) Detection of the primer extension product by SELDI-TOF Mass spectrometry**

The primer oligo base extension products (25 µl) were first mixed with 75 µl binding buffer and then applied to Q10 anionic chips (Vermillion, Inc) assembled in a bioprocessor. For multiplexed genotyping, a volume of 25 µL was pooled together from each of the oligonucleotide extension reactions for factor V Leiden, prothrombin G20210A, and MTHFR C677. The samples were then mixed with 75 µL binding buffer prior to applying to Q10 chip. After incubation for 30 minutes at room temperature, the chips were washed by a buffer containing 50 mM Tris, pH 7.0, three times. One µl of matrix containing 0.25M 3-Hydroxypicolinic acid (3-HPA) and 0.03M diammonium citrate in 10 % acetonitrile was added to each spot on the chip, and allowed to air dry. Finally, the chips were analyzed by PCS4000 SELDI-TOF mass spectrometer (Vermillion, Inc). All analytes genotyped by SELDI-TOF were sequenced to confirm accuracy of the analyte. Bidirectional sequencing was carried out by the Nucleic Acid Shared Resource of the Comprehensive Cancer Center, the Ohio State University (http://www.osuccc.osu.edu/nucleicacid/) using specific primers both up and downstream of each SNP site.

Clinical validation was performed by genotyping three SNPs (factor V Leiden, prothrombin G20210A, and MTHFR C677T) in clinical samples using SELDI-TOF. The results were then compared to the results obtained using the PCR-RFPL method from Clinical Laboratories at the Ohio State University Medical Center.

**(iv) Results**

By using factor V Leiden as an example, Figure 7 illustrates the principle by which a SNP is genotyped by a mass spectrometer in this embodiment. The DNA containing the SNP factor V Leiden (G1691A) was first amplified by a PCR reaction. The amplified product is then used as template for primer based extension to give rise to the DNA fragments with specific lengths corresponding to their genotypes. Afterwards, the genotypes were determined by mass spectrometer based on the masses of PCR products.

Optimization of the test conditions is essential to achieve test precision, detection sensitivity, and test reliability. First, various conditions for the PCR reactions, including the number of amplification cycles, annealing temperature, and extension time were optimized (data not shown). This enables the maximization of the PCR product (analyte) and at the same time reduces the test turnaround time. Afterwards, efforts were made to optimize the conditions for the detection process. For a typical readout by a conventional mass spectrometer such as Matrix-assisted laser desorption/ionization time of flight (MALDI-TOF), approximately 1 µl of sample can be applied to a plate. After the sample has dried out on the plate, the matrix is added followed by readout on a mass spectrometer. In contrast, the bioprocessor in SELDI-TOF allows for loading as much as 500 µl of sample to be concentrated on a chip surface. The bound sample can then be subjected to a clean-up in order to remove unwanted components before detection. Figure 8 demonstrates the benefits of the on-chip sample enrichment and clean-up by SELDI-TOF in comparison to a condition mimicking the performance by a conventional mass spectrometer. The PCR product containing factor V Leiden was measured under 4 different conditions on Q10 chips. Two readouts detect the analyte derived from 1 µl PCR sample, the loading volume mimicking the capacity when detected by a conventional mass spectrometer such as MALDI-TOF. In order to examine the on-chip clean-up effect, one of these two readouts was subjected to washing (50 mM Tris HCl, pH 7.0) prior to detection. The other two readouts were obtained after on-chip enrichment of 20 µl PCR products, with and without an additional step of washing. The results demonstrate that SELDI-TOF offers remarkable sample concentrating properties, greatly improving test detection capability. It is also apparent that a washing step is necessary in both cases to enhance signal to noise ratios. It is likely that the washing step after sample is loaded onto the plate removes interfering substances in the PCR products, hence greatly enhancing detection of PCR products.

Next, we determined the optimal sample loading volume, pH buffer, and washing condition for detection of factor V Leiden genotype by SELDI-TOF mass spectrometer. As shown in Figure 9, panel A shows the ability of Q10 chips to capture PCR product containing factor V Leiden (G1691A) in a dose-dependent manner. Various volumes of the PCR products were applied to the Q10 chips and the appropriate washing steps performed. The amount of the analyte detected by SELDI-TOF corresponds to the amount loaded onto the chip until the binding capacity of the chip is reached. Panel B illustrates the pH dependency for the binding of the analyte on the Q10 chip. The PCR product (20 µl) was loaded onto each of the Q10 chips using a bioprocessor. After a 30 minute binding reaction, Q10 Chips were washed 3 times, each with a buffer of a different pH. The data demonstrated that the buffer with a pH of 7 offers the optimal binding of analyte to Q10 Chips with respect to the signal/noise ratios and peak readouts, while the buffers with either alkaline or acidic pH conditions significantly reduced the binding of analytes to the chip in a pH dependent manner. Panel C shows the effect of various salt concentrations on the measurement of DNA analyte by SELDI-TOF. After the step of on-chip sample enrichment, we washed the chips using washing buffers with different concentration of NaCl as indicated. The buffer with lowest salt concentration gives rise to the cleanest analyte peak. When a high concentration of salt is used, the Na⁺ adducts, a cluster of side peaks with a difference of about 20 Daltons in molecular weight are seen on the right side of the analyte peak.

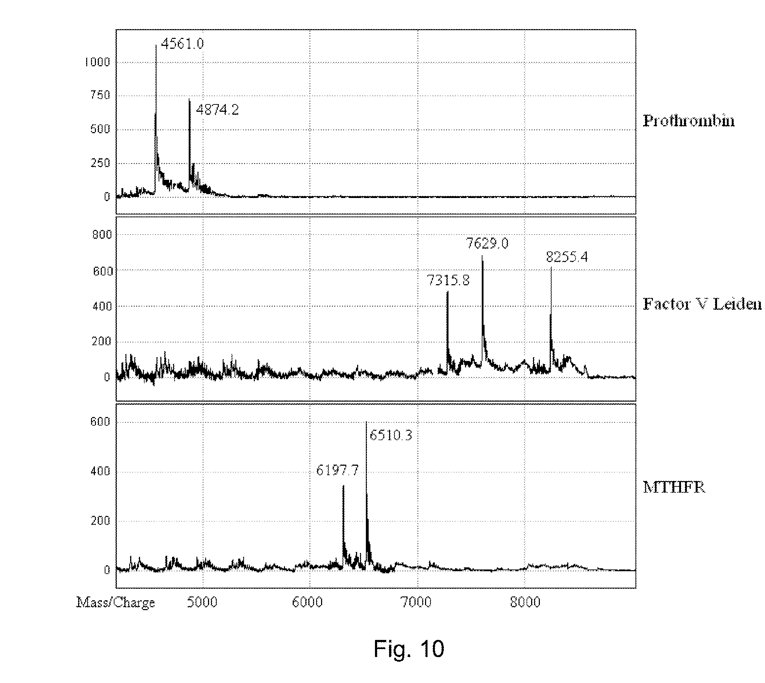

By using the optimized conditions for PCRs and the detection process, all known SNPs that predispose patients to thrombosis, factor V Leiden, prothrombin G20210A, and MTHFR C677T, were adequately detected by SELDI-TOF. The representative mass spectrums for these three SNPs are shown in Figure 10. The masses of the peaks for each of the three SNPs correspond well to the theoretical masses shown in Table 5. For instance, the middle panel in Figure 10 determines the genotype of the SNP at Factor V (G1691A), SELDI-TOF detected three peaks with masses of 7315.8Da 7629.0 and 8255.4Da respectively. Based on Table 5, the genotype for this individual is GA heterozygous, factor V Leiden. Similarly, the genotypes for this individual at the SNPs for prothrombin G20210A, and MTHFR (C677T), are GG and CC respectively.

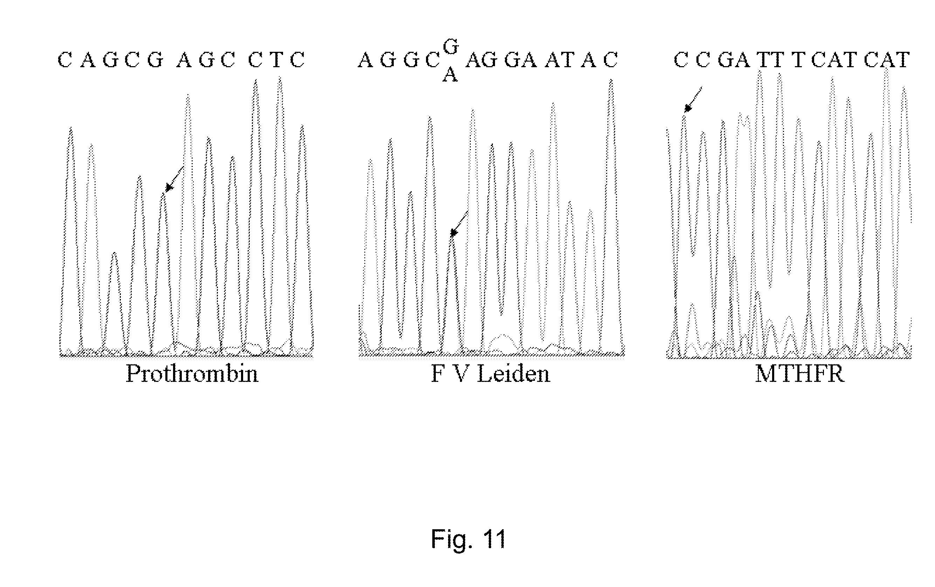

In order to evaluate the accuracy of the genotyping by SELDI-TOF, the amplified PCR products that were used for genotyping were also sequenced. As shown in Figure 11, the arrows point to the site of sequences that determine the genotype. For all SNPs of factor V Leiden, prothrombin G20210A, and MTHFR C677T, the results of sequencing agree with the genotype detected by SELDI-TOF.

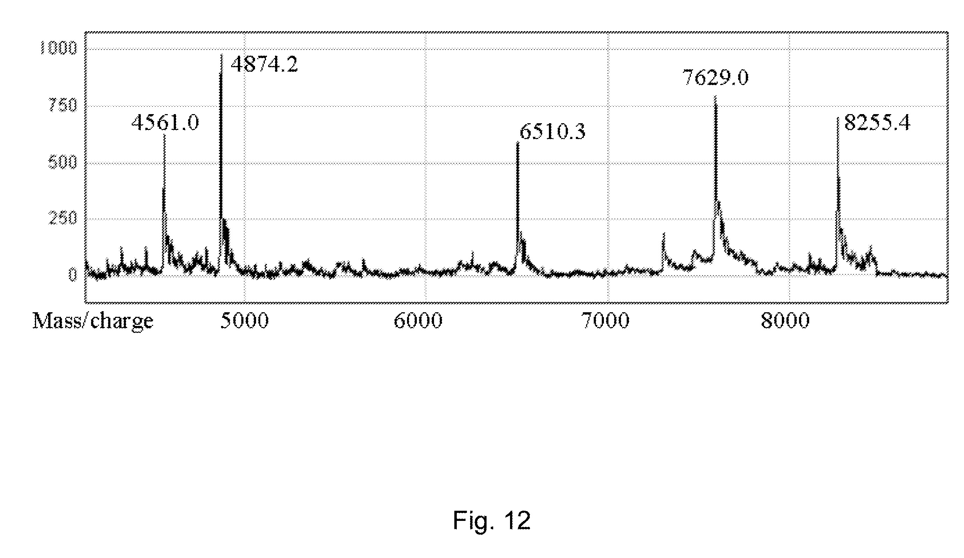

In order to improve the efficiency of genotyping by SELDI-TOF, we designed the genotyping primers of factor V Leiden, prothrombin G20210A, and MTHFR C677T with about 1000 Daltons of variance by adding different numbers of ddTTP to the 5' end of the primers (see Table 5). As shown in Figure 12, all three primer extension products detected separately in Figure 10 were loaded onto the same spot on SELDI chip, and then analyzed by mass spectrometer. The results show that the peaks that correspond to different genotypes are all displayed distinctly. Since these three SNPs can be detected simultaneously, the test turnaround time has been reduced to less than five hours.

Finally, the test accuracy by SELDI-TOF has been validated clinically. Among the 100 clinical samples that were submitted for determination of SNPs (40 for factor V Leiden, 40 for prothrombin G20210A, and 20 for MTHFR C677T), the genotyping tests were performed both in our laboratory by SELDI-TOF and in the clinical reference laboratory at the Ohio State University Medical Center using PCR-Restriction fragment length polymorphism (RFLP). The agreement rates in genotypes were 100% for all three SNPs. Additionally, we selected four clinical samples that were genotyped for all three SNPs (factor V Leiden, prothrombin G20210A, and 20 for MTHFR C677T) in the clinical reference laboratory. These samples were at least heterozygous for two of the three thrombophiliac SNPs. We genotyped these samples using multiplexed detection by SELDI-TOF. The results are same as reported by the clinical reference laboratory. The data demonstrates that multiplexed detection process by SELDI-TOF enables an accurate determination of SNP genotypes.

**C. Genotyping other genetic polymorphisms on SELDI-TOF platform.**

Other types of genetic polymorphisms may be detected using methods described herein. For example, a detection scheme for detecting polymorphisms that impact pharmacological performance of three cancer drugs are described below.

**(i) Detection of CYP2D6*3 (A2549 del) by SELDI-TOF**

One genetic polymorphism, CYP2D6*3 involves a single nucleotide deletion in the gene for CYP2D6. This polymorphism is known to be associated with inadequate responses to Tamoxifen. The frequency of this deletion is greater than 1% in the general population.

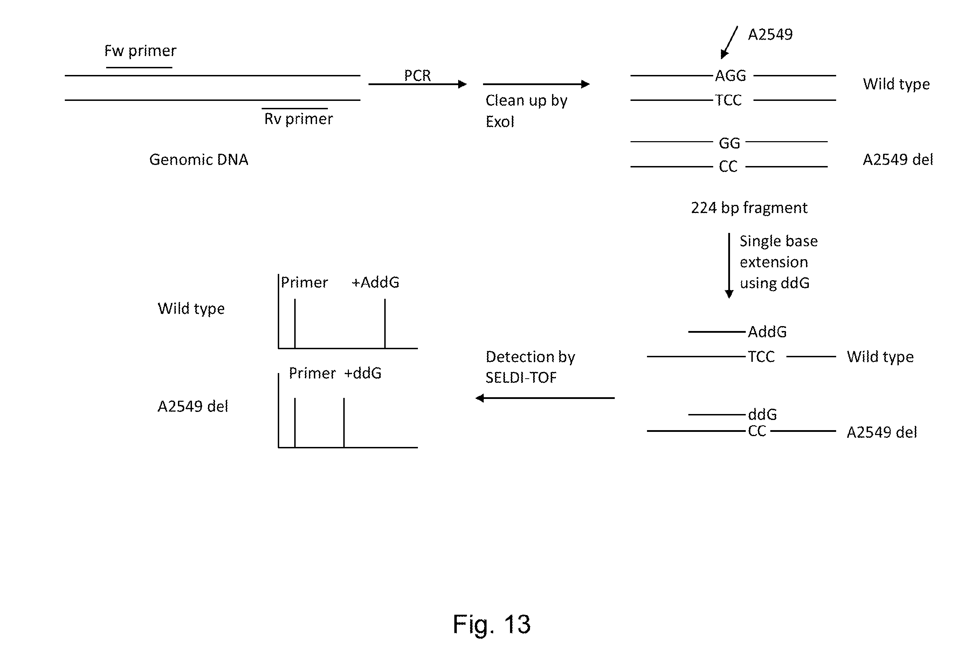

Figure 13 depicts an embodiment for a SELDI-TOF genotyping assay for the CYP2D6 *3 variant. Briefly, PCR may be performed to amplify the DNA fragment containing CYP2D6*3 SNP in the gene for CYP2D6. Afterwards, Exonuclease I is used to remove the excessive primers and dNTPs. Then single base extension (SBE) is conducted using a specific primer immediately upstream of the deletion site. Finally, single pair extension product is detected by SELDI-TOF to determine the genotype at this polymorphic site (wild type vs. A2549 deletion).

**(ii) Detection of UGT1A1 TA repeats by SELDI-TOF**

Embodiments may be used to detect essentially any allele specific sequence variation. For example, certain genetic polymorphisms comprise differences in the number of TA repeats. Such polymorphisms have been shown, for example, in the promoter region in the gene for the uridine diphosphate glucuronosyltransferase 1A1 (UGT1A1) enzyme. These polymorphisms cause a decreased expression of UGT1A1 enzyme, therefore an increased risk of Irinotecan associated drug toxicity.

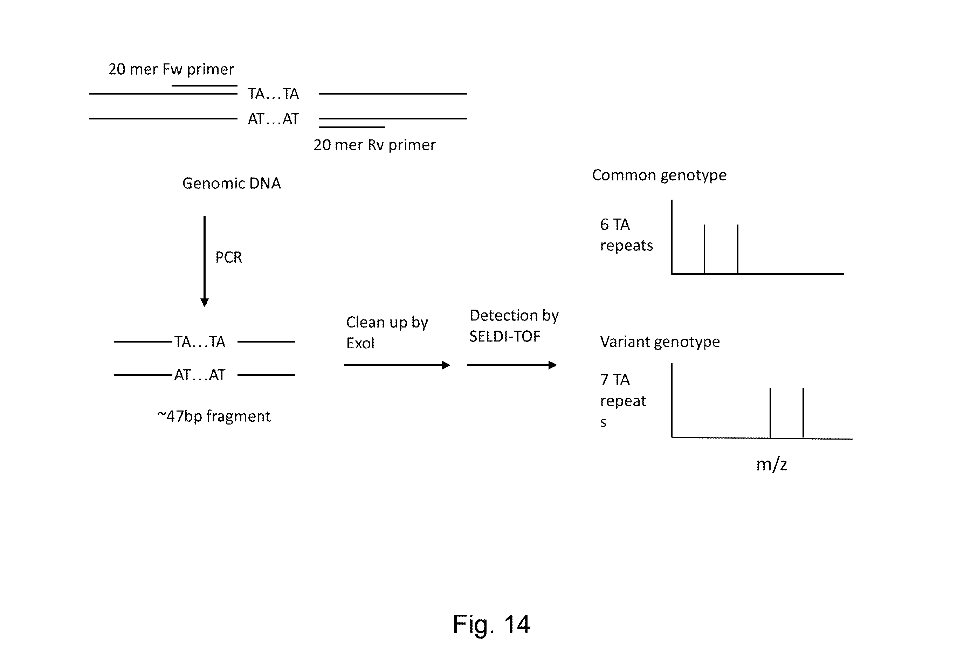

Figure 14 depicts an embodiment for genotyping wild type (containing 6 TA repeats) and a variant containing 7 TA repeats. In various embodiments, primers immediately up- and downstream of the TA repeat region may be used to perform a PCR reaction. The PCR amplified products may be adequately detected by SELDI-TOF. The genetic polymorphism contains 7 TA repeats, differing in Mᵣ from 6 TA repeats in the wild type. Therefore, the polymorphism may be genotyped using SELDI-TOF mass spectrometry.

References

The following documents are referred to:
1. Frazer KA, et al.: A second generation human haplotype map of over 3.1 million SNPs, Nature 2007, 449:851-861
2. Kim S, Misra A: SNP genotyping: technologies and biomedical applications, Annu Rev Biomed Eng 2007, 9:289-320
3. Tost J, Gut IG: Genotyping single nucleotide polymorphisms by MALDI mass spectrometry in clinical applications, Clin Biochem 2005, 38:335-350
4. Humeny A, Bonk T, Berkholz A, Wildt L, Becker CM: Genotyping of thrombotic risk factors by MALDI-TOF mass spectrometry, Clin Biochem 2001, 34:531-536
5. Hung K, Sun X, Ding H, Kalafatis M, Simioni P, Guo B: A matrix-assisted laser desorption/ionization time-of-flight based method for screening the 1691 G --> A mutation in the factor V gene, Blood Coagul Fibrinolysis 2002, 13:117-122
6. Tang N, Tornatore P, Weinberger SR: Current developments in SELDI affinity technology, Mass Spectrom Rev 2004, 23:34-44
7. Furie B, Furie BC: Molecular basis of vitamin K-dependent gamma-carboxylation, Blood 1990, 75:1753-1762
8. Gage BF, Lesko LJ: Pharmacogenetics of warfarin: regulatory, scientific, and clinical issues, J Thromb Thrombolysis 2008, 25:45-51
9. Wadelius M, Pirmohamed M: Pharmacogenetics of warfarin: current status and future challenges, Pharmacogenomics J 2007, 7:99-111
10. Yin T, Miyata T: Warfarin dose and the pharmacogenomics of CYP2C9 and VKORC1 - rationale and perspectives, Thromb Res 2007, 120:1-10
11. Zhu Y, Shennan M, Reynolds KK, Johnson NA, Herrnberger MR, Valdes R, Jr., Linder MW: Estimation of warfarin maintenance dose based on VKORC1 (-1639 G>A) and CYP2C9 genotypes, Clin Chem 2007, 53:1199-1205
12. Klein TE, Altman RB, Eriksson N, Gage BF, Kimmel SE, Lee MT, Limdi NA, Page D, Roden DM, Wagner MJ, Caldwell MD, Johnson JA: Estimation of the warfarin dose with clinical and pharmacogenetic data, N Engl J Med 2009, 360:753-764
13. Schwarz UI, Ritchie MD, Bradford Y, Li C, Dudek SM, Frye-Anderson A, Kim RB, Roden DM, Stein CM: Genetic determinants of response to warfarin during initial anticoagulation, N Engl J Med 2008, 358:999-1008
14. Meckley LM, Wittkowsky AK, Rieder MJ, Rettie AE, Veenstra DL: An analysis of the relative effects of VKORC1 and CYP2C9 variants on anticoagulation related outcomes in warfarin-treated patients, Thromb Haemost 2008, 100:229-239
15. Lenzini PA, Grice GR, Milligan PE, Dowd MB, Subherwal S, Deych E, Eby CS, King CR, Porche-Sorbet RM, Murphy CV, Marchand R, Millican EA, Barrack RL, Clohisy JC, Kronquist K, Gatchel SK, Gage BF: Laboratory and clinical outcomes of pharmacogenetic vs. clinical protocols for warfarin initiation in orthopedic patients, J Thromb Haemost 2008, 6:1655-1662
16. Ross P, Hall L, Smirnov I, Haff L: High level multiplex genotyping by MALDI-TOF mass spectrometry, Nat Biotechnol 1998, 16:1347-1351
17. Paracchini S, Arredi B, Chalk R, Tyler-Smith C: Hierarchical high-throughput SNP genotyping of the human Y chromosome using MALDI-TOF mass spectrometry, Nucleic Acids Res 2002, 30:e27
18. Misra A, Hong JY, Kim S: Multiplex genotyping of cytochrome p450 single-nucleotide polymorphisms by use of MALDI-TOF mass spectrometry, Clin Chem 2007, 53:933-939
19. Takahashi H, Wilkinson GR, Caraco Y, Muszkat M, Kim RB, Kashima T, Kimura S, Echizen H: Population differences in S-warfarin metabolism between CYP2C9 genotype-matched Caucasian and Japanese patients, Clin Pharmacol Ther 2003, 73:253-263
20. Landefeld CS, Beyth RJ: Anticoagulant-related bleeding: clinical epidemiology, prediction, and prevention, Am J Med 1993, 95:315-328
21. Gage B, Eby C, Johnson J, Deych E, Rieder M, Ridker P, Milligan P, Grice G, Lenzini P, Rettie A, Aquilante C, Grosso L, Marsh S, Langaee T, Farnett L, Voora D, Veenstra D, Glynn R, Barrett A, McLeod H: Use of Pharmacogenetic and Clinical Factors to Predict the Therapeutic Dose of Warfarin, Clin Pharmacol Ther 2008,
22. Wadelius M, Chen LY, Lindh JD, Eriksson N, Ghori MJ, Bumpstead S, Holm L, McGinnis R, Rane A, Deloukas P: The largest prospective warfarin-treated cohort supports genetic forecasting, Blood 2008,
23. King CR, Porche-Sorbet RM, Gage BF, Ridker PM, Renaud Y, Phillips MS, Eby C: Performance of commercial platforms for rapid genotyping of polymorphisms affecting warfarin dose, Am J Clin Pathol 2008, 129:876-883
24. Chace DH, Kalas TA, Naylor EW: Use of tandem mass spectrometry for multianalyte screening of dried blood specimens from newborns, Clin Chem 2003, 49:1797-1817
25. Dooley KC: Tandem mass spectrometry in the clinical chemistry laboratory, Clin Biochem 2003, 36:471-481
26. Venditti LN, Venditti CP, Berry GT, Kaplan PB, Kaye EM, Glick H, Stanley CA: Newborn screening by tandem mass spectrometry for medium-chain Acyl-CoA dehydrogenase deficiency: a cost-effectiveness analysis, Pediatrics 2003, 112:1005-1015
27. Saugy M, Cardis C, Robinson N, Schweizer C: Test methods: anabolics, Baillieres Best Pract Res Clin Endocrinol Metab 2000, 14:111-133
28. Chace DH, DiPerna JC, Naylor EW: Laboratory integration and utilization of tandem mass spectrometry in neonatal screening: a model for clinical mass spectrometry in the next millennium, Acta Paediatr Suppl 1999, 88:45-47
29. Keevil BG, Tierney DP, Cooper DP, Morris MR, Machaal A, Yonan N: Simultaneous and rapid analysis of cyclosporin A and creatinine in finger ##### blood samples using liquid chromatography tandem mass spectrometry and its application in C2 monitoring, Ther Drug Monit 2002, 24:757-767
30. Pieri M, Miraglia N, Castiglia L, Genovese G, Basilicata P, Simonelli A, Acampora A: Determination of rapamycin: quantification of the sodiated species by an ion trap mass spectrometer as an alternative to the ammoniated complex analysis by triple quadrupole, Rapid Commun Mass Spectrom 2005, 19:3042-3050
31. Maurer HH: Multi-analyte procedures for screening for and quantification of drugs in blood, plasma, or serum by liquid chromatography-single stage or tandem mass spectrometry (LC-MS or LC-MS/MS) relevant to clinical and forensic toxicology, Clin Biochem 2005, 38:310-318
32. Eichhorst J, Etter M, Lepage J, Lehotay DC: Urinary screening for methylphenidate (Ritalin) abuse: a comparison of liquid chromatography-tandem mass spectrometry, gas chromatography-mass spectrometry, and immunoassay methods, Clin Biochem 2004, 37:175-183
33. Deventer K, Van Eenoo P, Delbeke FT: Screening for amphetamine and amphetamine-type drugs in doping analysis by liquid chromatography/mass spectrometry, Rapid Commun Mass Spectrom 2006, 20:877-882
34. Scott SA, Edelmann L, Kornreich R, Desnick RJ: Warfarin pharmacogenetics: CYP2C9 and VKORC1 genotypes predict different sensitivity and resistance frequencies in the Ashkenazi and Sephardi Jewish populations, Am J Hum Genet 2008, 82:495-500
35. Bezemer ID, Bare LA, Doggen CJ, Arellano AR, Tong C, Rowland CM, Catanese J, Young BA, Reitsma PH, Devlin JJ, Rosendaal FR: Gene variants associated with deep vein thrombosis, Jama 2008, 299:1306-1314
36. Aiach M, Borgel D, Gaussem P, Emmerich J, Alhenc-Gelas M, Gandrille S: Protein C and protein S deficiencies, Semin Hematol 1997, 34:205-216
37. Vinazzer H: Hereditary and acquired antithrombin deficiency, Semin Thromb Hemost 1999, 25:257-263
38. Coppens M, van Mourik JA, Eckmann CM, Buller HR, Middeldorp S: Current practise of testing for inherited thrombophilia, J Thromb Haemost 2007, 5:1979-1981
39. Almawi WY, Tamim H, Kreidy R, Timson G, Rahal E, Nabulsi M, Finan RR, Irani-Hakime N: A case control study on the contribution of factor V-Leiden, prothrombin G20210A, and MTHFR C677T mutations to the genetic susceptibility of deep venous thrombosis, J Thromb Thrombolysis 2005, 19:189-196
40. McColl MD, Chalmers EA, Thomas A, Sproul A, Healey C, Rafferty I, McWilliam R, Eunson P: Factor V Leiden, prothrombin 20210G-->A and the MTHFR C677T mutations in childhood stroke, Thromb Haemost 1999, 81:690-694
41. Simioni P: Who should be tested for thrombophilia? Curr Opin Hematol 2006, 13:337-343
42. Whitlatch NL, Ortel TL: Thrombophilias: when should we test and how does it help? Semin Respir Crit Care Med 2008, 29:25-39
43. Xu B, Tubbs RR, Kottke-Marchant K: Molecular genetic testing of polymorphisms associated with venous thrombosis: a review of molecular technologies, Diagn Mol Pathol 2005, 14:193-202
44. Meyer K, Fredriksen A, Ueland PM: High-level multiplex genotyping of polymorphisms involved in folate or homocysteine metabolism by matrix-assisted laser desorption/ionization mass spectrometry, Clin Chem 2004, 50:391-402
45. Hulot JS, Bura A, Villard E, Azizi M, Remones V, Goyenvalle C, Aiach M, Lechat P, Gaussem P: Cytochrome P450 2C19 loss-of-function polymorphism is a major determinant of clopidogrel responsiveness in healthy subjects, Blood 2006, 108:2244-2247
46. Simon T, Verstuyft C, Mary-Krause M, Quteineh L, Drouet E, Meneveau N, Steg PG, Ferrieres J, Danchin N, Becquemont L: Genetic determinants of response to clopidogrel and cardiovascular events, N Engl J Med 2009, 360:363-375
47. Mega JL, Close SL, Wiviott SD, Shen L, Hockett RD, Brandt JT, Walker JR, Antman EM, Macias W, Braunwald E, Sabatine MS: Cytochrome p-450 polymorphisms and response to clopidogrel, N Engl J Med 2009, 360:354-362
48. Jin M, Cataland SR, Bissell M, Wu HM: A rapid test for the diagnosis of thrombotic thrombocytopenic purpura using surface enhanced laser desorption/ionization time-of-flight (SELDI-TOF)-mass spectrometry, J Thromb Haemost. 2006, 4:333-338.
49. Zhang Z, Bast RC, Jr., Yu Y, Li J, Sokoll LJ, Rai AJ, Rosenzweig JM, Cameron B, Wang YY, Meng XY, Berchuck A, Van Haaften-Day C, Hacker NF, de Bruijn HW, van der Zee AG, Jacobs IJ, Fung ET, Chan DW: Three biomarkers identified from serum proteomic analysis for the detection of early stage ovarian cancer, Cancer Res 2004, 64:5882-5890
50. Kearon C, Crowther M, Hirsh J: Management of patients with hereditary hypercoagulable disorders, Annu Rev Med 2000, 51:169-185
51. Den Heijer M, Lewington S, Clarke R: Homocysteine, MTHFR and risk of venous thrombosis: a meta-analysis of published epidemiological studies, J Thromb Haemost 2005, 3:292-299
52. Eldibany MM, Caprini JA: Hyperhomocysteinemia and thrombosis: an overview, Arch Pathol Lab Med 2007, 131:872-884
53. Hunt BJ, Shannon M, Bevan D, Murday V: Is a nihilistic attitude to thrombophilia screening justified? Thromb Haemost 2002, 87:918
54. Lowe G, Woodward M, Vessey M, Rumley A, Gough P, Daly E: Thrombotic variables and risk of idiopathic venous thromboembolism in women aged 45-64 years. Relationships to hormone replacement therapy, Thromb Haemost 2000, 83:530-535
55. US Patent Application Publication No. 2004/0038234

It is to be understood that while embodiments have been described in conjunction with the detailed description thereof, the foregoing description is intended to illustrate and not limit the scope of the invention.

## Claims

1. A method for rapidly genotyping a SNP marker in a DNA sample, comprising the steps of:
(a) providing a DNA sample;
(b) amplifying a segment of the DNA comprising the SNP marker using a primer pair;
(c) performing an oligonucleotide extension using a SNP-specific primer to generate an analyte mixture comprising an allele specific analyte;
(d) contacting the analyte mixture to a desorption spectrometry target having a chemical affinity to the allele specific analyte;
(e) washing the target with an eluant;
(d) detecting retained analyte on the target by desorption spectrometry; and
(e) identifying a genotype for the SNP marker in the DNA sample.

2. The method of claim 1, wherein the analyte comprises an oligonucleotide or other nucleic acid.

3. The method of claim 1 or 2, wherein the DNA sample is obtained from a specimen selected from the group consisting of tissue, blood, urine, stool, lymph, cerebrospinal fluid, saliva, buccal swab, and interarticular fluid.

4. The method of claim 1 wherein the DNA sample is obtained from a pathological cell.

5. The method of any of claims 1 to 4 wherein the detecting step is performed with a SELDI mass spectrometer.

6. A method for rapidly determining a genotype for a SNP marker of a subject, comprising the steps of:
(a) providing a specimen comprising genomic DNA from the subject;
(b) amplifying a segment of the DNA comprising the SNP marker using a primer pair;
(c) performing an oligonucleotide extension using a SNP-specific primer to generate an analyte mixture comprising an allele specific analyte;
(d) contacting the analyte mixture to a desorption spectrometry target having a chemical affinity to the analyte;
(e) washing the target with an eluant;
(d) detecting retained analyte on the target by desorption spectrometry; and
(e) identifying the genotype for the SNP marker of the subject based on the mass and charge of the analyte.

7. The method of claim 6, wherein the analyte comprises an oligonucleotide.

8. The method of claim 1 or 6, wherein the desorption spectrometry target comprises an adsorbent array.

9. The method of claim 8, wherein the adsorbent array comprises a protein chip.

10. The method of claim 8, wherein the adsorbent array is selected from the group consisting of a hydrophobic adsorbent, a thiophilic adsorbent, an ion exchange adsorbent, a metal ion adsorbent, or antibody affinity matrices.

11. The method of any one of claims 1, 2 or 6 to 10, wherein the SNP marker is associated with a disease selected from the group consisting of cancer, cardiovascular disease, thrombotic diseases, autoimmune disease, viral infection, Alzheimer's disease, and diabetes.

12. The method of any of claims 6 or 7, wherein the specimen is obtained from a specimen selected from the group consisting of tissue, blood, urine, stool, lymph, cerebrospinal fluid, saliva, buccal swab, and interarticular fluid.

13. A kit comprising:
a target having a chemical affinity toward oligonucleotides or other nucleic acids;
a DNA polymerase; and
instructions for performing the method of claim 1.

14. A method for DNA genotyping a sequence polymorphism of a subject by SELDI-TOF mass spectrometry, comprising the steps of:
(a) providing a specimen comprising genomic DNA from the subject;
(b) generating from the DNA an allele specific analyte in a reaction mixture;
(c) contacting the reaction mixture to a desorption spectrometry target having a chemical affinity to the allele specific analyte;
(d) washing the target with an eluant to purify the allele specific analyte;
(e) detecting retained analyte on the target by desorption spectrometry; and
(f) identifying the genotype for the sequence polymorphism of the subject based on the mass and charge of the allele specific analyte.


SEQUENCE LISTING

<110>  The Ohio State University
       Wu, Haifeng
       Yang, Shangbin
       Xu, Lihui

<120>  RAPID GENOTYPING OF SNPs

<130>  OSU1159-258

<150>  US 61/157,812
<151>  2009-03-05

<160>  18

<170>  PatentIn version 3.5

<210>  1
<211>  20
<212>  DNA
<213>  Artificial Sequence

<220>
<223>  Primer

<400>  1
gggtaggtgc aacagtaagg                                                   20


<210>  2
<211>  20
<212>  DNA
<213>  Artificial Sequence

<220>
<223>  Primer

<400>  2
tttggactac aggtgcctgc                                                   20


<210>  3
<211>  20
<212>  DNA
<213>  Artificial Sequence

<220>
<223>  Primer

<400>  3
ccctcatgac gctgcggaat                                                   20


<210>  4
<211>  20
<212>  DNA
<213>  Artificial Sequence

<220>
<223>  Primer

<400>  4
gaaagatatg gccacccctg                                                   20


<210>  5
<211>  20
<212>  DNA
<213>  Artificial Sequence

<220>
<223>  Primer

<400>  5
catgcaagac aggagccaca                                                   20


<210>  6
<211>  20
<212>  DNA
<213>  Artificial Sequence

<220>
<223>  Primer

<400>  6
tctctcaccc ggtgatggta                                                   20


<210>  7
<211>  15
<212>  DNA
<213>  Artificial Sequence

<220>
<223>  primer for oligonucleotide extension

<400>  7
aaacaaccat tggcc                                                        15


<210>  8
<211>  18
<212>  DNA
<213>  Artificial Sequence

<220>
<223>  primer for oligonucleotide extension

<400>  8
gggcttcctc ttgaacac                                                     18


<210>  9
<211>  20
<212>  DNA
<213>  Artificial Sequence

<220>
<223>  primer for oligonucleotide extension

<400>  9
gctggtgggg agaaggtcaa                                                    20


<210>  10
<211>  20
<212>  DNA
<213>  Artificial Sequence

<220>
<223>  Primer

<400>  10
atggggtgaa ggctgtgacc                                                    20


<210>  11
<211>  18
<212>  DNA
<213>  Artificial Sequence

<220>
<223>  primer

<400>  11
agcactggga gcattgag                                                      18


<210>  12
<211>  20
<212>  DNA
<213>  Artificial Sequence

<220>
<223>  primer

<400>  12
acatcgcctc tgggctaata                                                    20


<210>  13
<211>  20
<212>  DNA
<213>  Artificial Sequence

<220>
<223>  primer

<400>  13
ttgaaggaaa tgccccatta                                                    20


<210>  14
<211>  18
<212>  DNA
<213>  Artificial Sequence

<220>
<223>  primer

<400>  14

ccaaaggcca ccccgaag                                                    18

<210>  15
<211>  20
<212>  DNA
<213>  Artificial Sequence

<220>
<223>  primer

<400>  15
gaaagatccc ggggacgatg                                                  20

<210>  16
<211>  15
<212>  DNA
<213>  Artificial Sequence

<220>
<223>  primer for oligonucleotide extension

<400>  16
aaagtgactc tcagc                                                       15

<210>  17
<211>  24
<212>  DNA
<213>  Artificial Sequence

<220>
<223>  primer for oligonucleotide extension

<400>  17
tttttttttg atccctggac aggc                                             24

<210>  18
<211>  20
<212>  DNA
<213>  Artificial Sequence

<220>
<223>  primer for oligonucleotide extension

<400>  18
gctgcgtgat gatgaaatcg                                                  20